# EUROPEAN PATENT SPECIFICATION

(11) **EP 0 592 674 B1**
(45) Date of publication and mention of the grant of the patent: **29.11.2000**
(21) Application number: 93900380.2
(22) Date of filing: 24.12.1992
(51) Int. Cl.: G01N 33/84

(54) **METHOD OF ASSAYING METAL PRESENT IN BIOLOGICAL SPECIMEN AND REAGENT THEREFOR**
VERFAHREN ZUR BESTIMMUNG VON METALLEN IN BIOLOGISCHEN PROBEN UND REAGENZ DAFÜR
PROCEDE DE TITRAGE DE METAUX PRESENTS DANS DES ECHANTILLONS BIOLOGIQUES ET REACTIF RELATIF

(30) Priority: 25.12.1991 JP 35642391
(43) Date of publication of application: 20.04.1994
(73) Proprietor: IATRON LABORATORIES, INC., Chiyoda-ku Tokyo 101 (JP)
(72) Inventor: NISHIDATE, Kazuyoshi, Iatron Laboratories, Inc., Chiyoda-ku, Tokyo 101 (JP); SUZUKI, Hiroshi, Iatron Laboratories, Inc., Chiyoda-ku, Tokyo 101 (JP); SUZUKI, Youko, Iatron Laboratories, Inc., Chiyoda-ku, Tokyo 101 (JP); KOGA, Youko, Iatron Laboratories, Inc., Chiyoda-ku, Tokyo 101 (JP)
(74) Representative: Goddar, Heinz J., Dr.
(86) International application number: JP9201685
(87) International publication number: WO9313422

(56) References cited:
- EP-A- 0 387 784
- JP-A- 48 003 798
- JP-A- 59 023 252
- JP-A- 62 006 170
- US-A- 3 547 586
- BIOCHEMISTRY, vol. 18, no. 25, 1979 WASHINGTON DC USA, pages 5562-5566, F.M. RAUSHEL ET AL. 'Paramagnetic probes for carbamoyl-phosphate synthetase: metal ion binding studies and preparation of nitroxide spin-labelled derivatives.'

## Description

### Technical Field

The present invention relates to a method to measure metals in samples of body fluid, in which metals other than the metals of interest (objective metals of measurement) are added to the measurement system to release the metals of interest bound to co-existing substances in the sample from said co-existing substances, so that even trace metals can be measured with high accuracy.

### Background Art

In the field of clinical examination, the measurement of various kinds of metals in samples (components) of body fluid has frequently been used as index for disease diagnosis. For example, the measurement of magnesium as a diagnostic means for ischemic heart disease, hyerthyroidism, etc.; the measurement of iron as a diagnostic means for various kinds of anemia, chronic hepatopathy, etc.; the measurement of copper as a diagnostic means for disease of hepatic duct, anemia, etc.; the measurement of calcium as a diagnostic means for nephropyelitis, nephrosis, etc.; and the measurement of zinc as a diagnostic means for disease of circulatory organs, hemolytic anemia, etc. have usually been used. Other metals encountered in the living body including aluminum, nickel, manganese, chromium and so on have also been measured and used as indices of various kinds of diseases.

The typical methods of measurement of these metals are, for example, atomic absorption spectrometry, emission spectrochemical analysis, X-ray fluorescence analysis, voltammetry, chelatometry, and the like. However, these methods are inadequate, particularly for daily examinations in the clinical examination field, since not only they require special, expensive apparatuses but also pretreatments of samples in such methods are troublesome. Therefore, recently various other kinds of methods for measuring metals in body fluids and tissues have been developed. Among these, the methods for measuring major metals are, for example, the following:
(i) To measure magnesium, there have been disclosed colorimetric methods in which titan yellow and xylidyl blue are used as color-producing agents, and enzymatic methods which utilize the activation of an enzyme reaction, based on one of the physiological actions of magnesium (Japanese Patent Application Laid-open Nos. 124398/1986 and 30597/1989), and the like.
(ii) To measure iron, colorimetric methods have been frequently used, in which bathophenanthroline and tripyridil-triazine are used as color-producing agents after reduction of free trivalent iron ions into bivalent iron ions with reducing agents such as ascorbic acid and the like, and various kinds of other color-producing agents have been developed (Japanese Patent Application Laid-open Nos. 144750/1983 and 301678/1989).
(iii) To measure copper, colorimetric methods are disclosed, in which bathocuproinedisulfonic acid and the like are used as color-producing agents after addition of reducing agent, and in which co-existring metals are masked and the 2-pyridylazo-aminophenol derivatives are then used as the color-producing agents (Japanese Patent Application Laid-open No. 69552/1985), and so on.
(iv) To measure calcium, colorimetric methods are disclosed, in which σ-cresolphtalein complexone is used as a color-producing agent under alkaline condition. In addition, there has also been known the enzymatic method which utilizes the action of enzyme activity as an activating factor which is one of physiological actions of calcium (Japanese Patent Application Laid-open No. 142498/1990).
(v) To measure zinc, colorimetric methods are frequently used, in which zinc is reacted with [2-(5-bromo-2-pyridyl)azo]-5-(dimethylamino)phenol in the presence of surfactants to form a complex compound.

The samples to be tested for the presence of metals entail; blood (whole blood, blood serum, blood plasma, blood cell), urine, feces, hair, saliva, breast milk, and so on. Generally speaking, in clinical examination, metals contained in blood or urine are mainly measured.

In the measurement of metals, as indicated above, color-producing agents which specifically react (bond) with the objective metals (i.e., chelating agents) are generally used. In the above-mentioned enzymatic methods, the enzymes which are specifically acted upon by the metals of interest are chosen, and the existing amount of metals is determined quantitatively according to the variability of the enzyme activity values.

However, in measurements using enzymatic or colorimetric methods, which utilize the color-producing agents mentioned above, desirable data sometimes cannot be obtained.

That is, the formation of a complex between the objective metal of measurement and the color-producing agent or the physiological interaction of the objective metals and an enzyme is inhibited or interfered with by some unknown factor.

Various factors are considered as causes of such phenomena. One possibility is that the metals of interest in a sample bind to substances already present in or added to the sample.

For example, samples may contain substances derived from medicines containing components having a metal-binding capacity (e.g., eliminants of renal calculus, detoxifying agents, metal-eliminating agents, anti-coagulants, etc.). Other samples may contain substances derived from various kinds of agents used in blood sampling tubes, e.g. derivatives from ethylenediamine (e.g., ehtylenediaminetetraacetates, etc.), deferoxamine mesilate, citric acid, tartaric acid, etc.

These are just examples of substances which can bind to the metals of interest in samples of body fluid and many other substances can be included.

It is well known that sodium ethylenediaminetetraacetate (hereinafter, referred to as EDTA) can react with almost all metallic ions except monovalent metallic ions to form stable, water-soluble chelate complexes. However, when EDTA or other substances similar to EDTA are present in samples, they may form chelate complexes with the metals of interest, thus making an accurate measurement of the metals of interest impossible. Particularly, in cases where the substances bound to the metals of interest are present in the patients themselves, clinical examination including the measurement of metals may be influenced by these substances. Re-sampling and re-examination may be necessary, which may burden not only patients but also the clinical examination facility.

In the Japanese Patent Application JP-A-59-23252 a method for measuring low-molecular-weight-components, particularly thyroid hormone, contained in a sample derived from a living body, such as blood, serum, plasma, etc, while eliminating the inhibition by co-existing proteins as the presence of protein has a great influence on the results of measurements, the method is aimed to avoid such an influence of proteins by bonding the proteins to a blocker-compound.

The Japanese Patent Application JP-A-48-3798 discloses a method to measure calcium contained in serum, while eliminating the inhibitory action of coexisting phosphorus by adding a dye under alkaline conditions and determining the color development corresponding to the calcium content by colorimetry or determining the amount of fluorescence corresponding to the calcium content, wherein a salt of molybdic acid is previously added to the serum sample and reacted with phosphorus contained in the sample, thereby eliminating the influence of phosphorus on the color development or the generation of fluorescence. Such a method therefore aims to avoid the influence of phosphorus on the measurement.

In view of the foregoing, the present inventors have carried out extensive studies to provide a method for measuring metals in body fluids, in which the metals of interest can be measured with high accuracy even in presence of various interfering chelating substances which may greatly affect the measured values in the measurement of metals in samples of body fluids. As a result, the present invention has been completed.

### Disclosure of invention

The present invention is defined by independent claim 1, claim 2 describes a preferred method.

As mentioned above, substances able to bind to the metals of interest (hereinafter, referred to as interfering chelating substance) may be present in the samples and the metals of interest may be bound to it.

In such a sample, a sufficient reaction does not occur by addition of the reacting reagents of detection (e.g., color-producing substances) to the sample. As a result, colorimetric or enzymatic measurements of the metal of interest are inaccurate.

To solve this problem, metals other than the metals of interest are added to the sample. The latter also bind to the interfering chelating substances present in the sample and thus, via the principle of competition, free the metal of interest from these substances, thus enabling an accurate measurement of these metals.

Examples of the metals of interest include potassium, calcium, magnesium, zinc, manganese, aluminum, cadmium, chromium, mercury, iron, copper, lead, nickel, selenium, bismuth, arsenic, etc.

As mentioned above, recently, various kinds of metal indicator reagents specific to such metals have been developed, and the colorimetric measurement can be carried out easily. A part of embodiments of the metal indicator reagents are shown in the following:
(a) magnesium: xylidyl blue, δ-hydroxy-5-quinolinesulfonic acid;
(b) calcium: σ-cresolphtalein complexone;
(c) copper: bathocuproine, 2-[(5-bromo-2-pyridyl)azo]5-[N-propyl-N-(3-sulfopropyl)amino]phenol, 2-(2-thiazolylazo)-5-N-ethyl-N-sulfopropylamino benzoic acid (TSAB);
(d) zinc: ([2-(5-bromo-2-pyridyl)azo]-5-(diethylamino)phenol(5-Br-PADAP);
(e) iron: bathophenanthroline, tripyridil-triazine, ferrozine, σ-phenanthroline, 2-nitroso-5-(N-propyl-N-sulfopropylamino)-phenol (Nitroso-PSAP);
(f) aluminum: 1-(1-hydroxy-4-methyl-2-phenylazo)-2-naphtol-4-sulfonic acid;
(g) cadmium: α, β, γ, δ-tetraphenylporphine tetrasulfonate;
(h) chromium: 3,3'-di(N-methyl-N-carboxymethylaminomethyl)-σ-cresolsulfonphtalein;
(i) mercury: diphenyithiocarbazone;
(j) lead: diphenylthiocarbazone, 2,7-bis(4-methyl-2-sulfophenylazo)-1,8-dihydroxynaphthalene-3,6-disulfonic acid;
(k) nickel: 2-furildioxime; and
(l) selenium: 3,3-diaminobendizine.

Examples of enzymes used in the measurement of magnesium and calcium, in which the enzymatic reactions are applied, include glucokinase, hexokinase, amylase, glycerolkinase, isocitrate dehydrogenase, etc. These enzymes are derived from bacteria or yeast, but the origins of the enzymes are not limited in particular.

According to the present invention, metals other than the metals of interest, which have no reactivity or, if any, extremely little reactivity with the said color-producing metal indicator reagents, are arbitrarily selected and added to the measurement system.

For example, in ease where iron in blood serum is measured using 2-(5-nitro-2-pyridylazo)-5-(N-propyl-N-sulfopropylamino)-phenol (NPS) as a color-producing metal indicator reagent, metals other than iron, which never bind to NPS to develop color, are added to the measurement system. Examples of such metals are lanthanum, manganese, terbium, yttrium, ytterbium, palladium, gallium, strontium, aluminum, etc.

On the other hand, in cases where magnesium is measured by enzymatic methods, metals other than magnesium, which never inhibit the physiological action of enzymes such as hexokinase, glucokinase, glycerolkinase, etc. and magnesium (i.e., expression of enzyme activity), are added to the measurement system. Examples of such metals are: cobalt, aluminum, lanthanum, bismuth, nickel, etc., and many other metals may be used.

In addition, in cases where other metals such as zinc, copper, etc. are measured using color-producing metal indicator reagents, the present invention can be also applied according to the same method as mentioned above.

The amount of metals other than the metals of interest to be added to the reaction system, can be arbitrarily selected depending on the contents of interfering chelating substances and the metals of interest. Metals other than the metals of interest may be added in an amount at least enough to make the metals of interest free from the interfering chelating substance, i.e., enough to make occur the objective reaction with the color-producing metal indicator reagent or enzyme. The amount of the metals other than the metals of interest to be added should be adjusted according to amount and kinds of the metals of interest, the composition of the reaction system and the interfering chelating substances, as required. Although it cannot be said absolutely, the reagents containing metals other than the metals of interest in an amount in the range from 5 µM to 2 M, preferably 10 µM to 1 M, may be used in the measurement. The form of the metals other than the metals of interest to be added is preferably the salts thereof, and they may be prepared within the above concentration range.

In the composition of the reaction system, the color-producing indicator reagents and enzymes may be contained within the known content range, and they are used preferably by dissolving in the known buffer solutions, i.e., the buffer solutions able to buffer the pH range in which the color developing reaction of metal indicator reagents and the expression of enzyme reactions are possible (i.e., around the appropriate pH within pH 4 to 10), such as tris-hydrochloride buffer solution, acetate buffer solution, phosphate buffer solution, citrate buffer solution, Good's buffer solution, etc. The metals other than the metals of interest may co-exist in said solutions or may be prepared as another reagent. Said reagents may contain known preservatives, stabilizers, various kinds of surfactants, reductants or oxidants.

The reagents for enzymatic methods can be preserved for long periods by freeze-drying them using conventional means.

### Brief description of drawings

Fig. 1 (a) illustrates a graph which represents the magnesium content in blood serum determined with addition of aluminum as a metal other than the metal of the interest.
Fig. 1 (b) illustrates a graph which represents the magnesium content in blood serum determined with addition of cobalt as a metal other than the metal of the interest.
Fig. 2 illustrates a graph which represents the magnesium content in blood serum determined without addition of metal other than the metal of interest. (Comparative example to Fig. 1.)
Fig. 3 illustrates a graph which represents the magnesium content in blood serum determined with addition of nickel and bismuth as metals other than the metal of interest.
Fig. 4 (a) illustrates a graph which represents the iron content in blood serum determined with addition of lanthanum as a metal other than the metal of interest.
Fig. 4 (b) illustrates a graph which represents the iron content in blood serum determined with addition of manganese as a metal other than the metal of interest.
Fig. 5 illustrates a graph which represents the iron content in blood serum determined without addition of metal other than the metal of interest. (Comparative example to Fig. 4.)
Fig. 6 illustrates a graph which represents the copper content determined with addition of lanthanum as a metal other than the metal of interest.
Fig. 7 illustrates a graph which represents the zinc content determined with addition of lanthanum as a metal other than the metal of interest.

### Best Mode for Carrying Out of the Invention

The present invention is further illustrated in detail by the following examples.

### 〈Example 1〉

### Measurement of Magnesium

To a commercially available control blood serum (NESCALL XA: a trade name produced by Nippon Shoji Co., Ltd.) of the known magnesium content (2.8 mg/dl), EDTA·2Na was added as a interfering chelating substance to obtain samples of concentration of 40, 80, 120, 160 and 200 mg/dl, respectively. Then, the following reagents were prepared to measure the magnesium content of the samples.

### (Reagent 1)

100 mM Tris buffer solution which (pH 8.5) containing:

| | |
|---|---|
| ATP (adenosine triphosphate) | 10 mM |
| NADP (nicotinamide adenine dinucleotide phosphate) | 0.5 mM |
| Glucokinase | 0.35 U/ml |
| G6PDH (glucose-6-phosphate dehydrogenase) | 0.6 U/ml |
| GEDTA (glycoletherdiaminetetraacetic acid) | 0.4 mM. |

### (Reagent 2)

100 mM Tris buffer solution which containing 10 mM of glucose (pH 8.5).

### (Procedure)

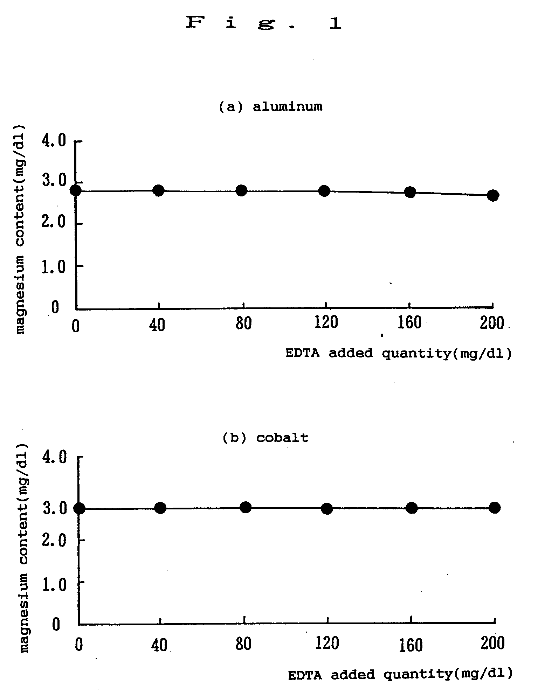

To 5 µl of each control blood serum containing EDTA, 400 µl of Reagent 1 in which each of aluminum and cobalt as a metal other than the objective metal of measurement was contained in a concentration of 300 µM, respectively, was added, followed by heating to 37°C. To the resulting reaction solution, 100 µl of Reagent 2 was added, and the reaction was then started at 37°C. The absorbance of each sample was measured at 340 nm of wave length 2 to 3 minutes after the reaction being started. At the same time, another procedure was carried out in the same manner as the above procedure except using purified water instead of the control blood serum, and the resulting value was defined as a blanc value. The magnesium content in each control blood serum with EDTA was calculated from the resulting absorbance value against that of the control blood serum without EDTA as a standard. Results are shown in Figs. 1 (a) and (b).

### 〈Comparative Example 1〉

The magnesium content in each control blood serum was calculated in the same manner as Example 1 by using Reagent 1 of the said Example 1 as it was (i.e., without additional metals). Results are shown in Fig. 2.

Apparently from the comparison between Figs. 1 (a) and (b) and Fig. 2, when metals other than magnesium (i.e., the objective metal of measurement) were added, the magnesium content can be measured accurately without influence of EDTA as a interfering chelating substance.

### 〈Example 2〉

The commercially available control blood serum of Example 1 was diluted two-fold with purified water (i.e., 1.4 mg/dl of magnesium content), and EDTA·2Na was then added thereto to give samples containing 10, 20, 30, 40 and 50 mg/dl in a concentration of EDTA·2Na, respectively. The magnesium content of each resulting sample was measured according to the same procedure as Example 1 using Reagents 1 and 2 of Example 1.

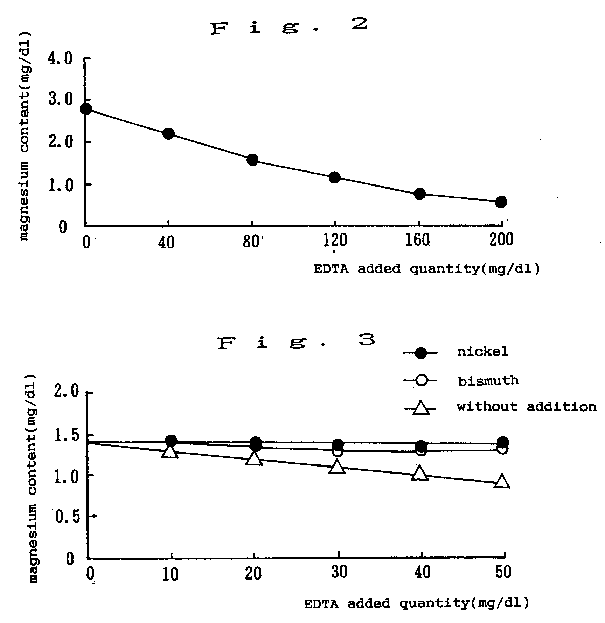

In this procedure, Reagent 1 to be used was one which was prepared by adding nickel and bismuth as additional metals in a concentration of 150 µM, respectively. Results are shown in Fig. 3. Apparently from Fig. 3, the effect of addition of metals other than magnesium is confirmed.

### 〈Example 3〉

### Measurement of Iron

To the commercially available control blood serum (Q-PAK: a trade name produced by Hiland Co., Ltd.) of known iron content (150 µg/dl), EDTA·2Na was added as a interfering chelating substance, to give samples containing 40, 80, 120, 160 and 200 mg/dl in concentration of EDTA·2Na, respectively. The resulting samples were measured iron content using the reagents prepared as follows:

### (Reagent 3)

0.5 M Acetate buffer solution containing 0.2 M of ascorbic acid (pH 4.0).

### (Reagent 4)

An aqueous solution in which 200 mg of NPS being dissolved in purified water to a total volume of 1000 ml.

### (Procedure)

To 100 µl of each control blood serum containing EDTA, 350 µl of Reagent 3 which containing each one of lanthanum, manganese, terbium, yttrium, ytterbium, palladium, gallium, indium, strontium and aluminum in concentration of 20 mM, respectively, was added, followed by heating to 37°C. 200 µl Of Reagent 4 was added thereto, and the reaction was then started at 37°C. The absorbance of each sample was measured at 590 nm of wave length 5 minutes after reaction being started. At the same time, another procedure was carried out in the same manner as the above procedure except using purified water instead of the control blood serum, and the resulting value was defined as a blanc value. The iron content in the each control blood serum with EDTA was calculated from the resulting absorbance value using the control blood serum without EDTA as a standard. Results obtained by using lanthanum and manganese as additional metals are shown in Figs. 4 (c) and (d). On the other hand, results obtained by using metals other than lanthanum and manganese as additional metals are shown in Table 1 below as measured values of iron content in the control blood serum containing 200 mg/dl of EDTA.

**Table 1**

| Metal | Iron Content (µg/dl) |
|---|---|
| Tb | 142.5 |
| Y | 146.7 |
| Yb | 143.3 |
| Pd | 148.1 |
| Ga | 150.8 |
| In | 147.7 |
| Sr | 142.0 |
| Al | 146.9 |

### 〈Comparative Example 2〉

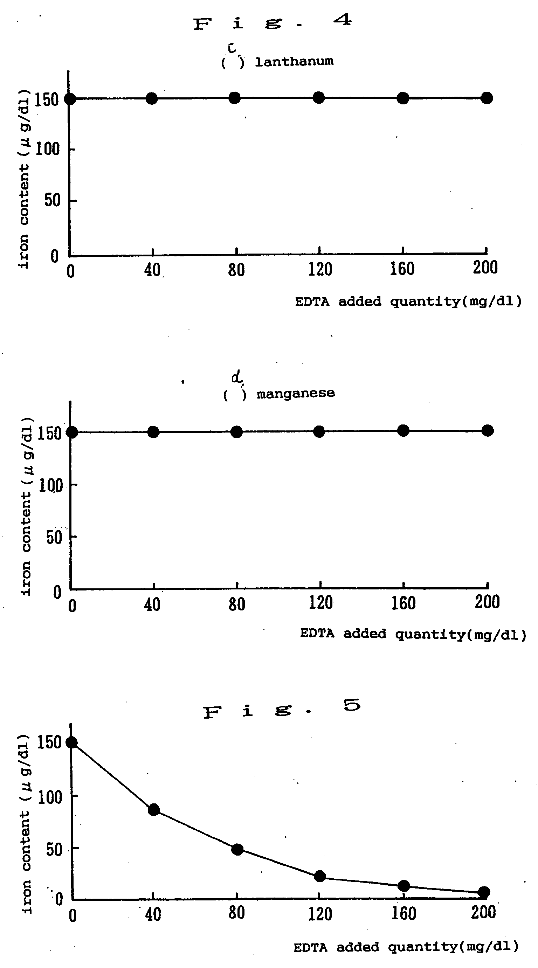

The iron content in each control blood serum was calculated in the same manner as Example 3 using Reagent 3 of the said Example 3 as it was (i.e., without additional metals). Results are shown in Fig. 5.

Apparently from the comparison between Figs. 4 (c), (d) and Table 1 and Fig. 5, when metals other than iron (i.e., the metal of interest) were added, the iron content can be measured accurately without influence of EDTA as a interfering chelating substance.

### 〈Example 4〉

### Measurement of Iron

A procedure was carried out in the same manner as Example 3 except using an aqueous solution in which 200 mg of Nitroso-PSAP was dissolved in purified water to a total volume of 1000 ml instead of Reagent 4 of said Example 3. In this procedure, the absorbance of each sample was measured at 760 nm of wave length. Results are shown in Table 2 below.

**Table 2**

| Metal | Iron Content (µg/dl) |
|---|---|
| La | 149.1 |
| Mg | 148.9 |
| Tb | 143.3 |
| Y | 145.2 |
| Yb | 144.1 |
| Pd | 146.3 |
| Ga | 147.6 |
| In | 148.9 |
| Sr | 144.4 |
| Al | 145.7 |

### 〈Example 5〉

### Measurement of Copper

7.9 mg Of copper sulfate 5-hydrate salt was dissolved to purified water to a total volume of 1000 ml, to give a standard solution of 200 µg/dl of copper concentration. The resulting solution was further diluted with purified water to give a dilution line of 25, 50, 100, 150 and 200 µg/dl of copper concentrations. To each member of the dilution line, EDTA·2Na salt was added as a interfering chelating substance in a concentration of 200 mg/dl. The copper content was then measured using the following reagents:

### (Reagent 5)

0.5 M Acetate buffer solution (pH 4.0).

### (Reagent 6)

An aqueous solution in which 200 mg of TSAB being dissolved in purified water to a total volume of 100 ml.

### (Reagent 7)

0.5 M Acetate buffer solution containing 30 mM of lanthanum (pH 4.0).

### (Procedure)

To 100 µl of each member of the dilution lines containing EDTA, 2.0 ml of Reagent 5 was added, and then heated to 37°C. Then, 500 µl of Reagent 6 was further added to the resultant. After the reaction of the resulting solution being carried out at 37°C for 5 minutes, the absorbance of each sample at 640 nm of wave length was measured.

Another test was carried out according to the same manner as the above procedure except using Reagent 7 instead of Reagent 5. Further another procedure was also carried out in the same manner as the above procedure except using purified water instead of the dilution line, and the resulting value was defined as a blank value. Results are shown in Fig. 6.

According to the Fig. 6, the method of the present invention apparently can measure the copper content more accurately compared to the conventional methods.

### 〈Example 6〉

### Measurement of Zinc

2.0 mg Of zinc powder was weighed accurately, and then 25 µl of hydrochloric acid was added thereto, followed by dissolving in purified water to a total volume of 1000 ml, to give a standard solution of 200 µg/dl of zinc concentration. The resulting solution was further diluted with purified water to give a dilution line of 25, 50, 150 and 200 µg/dl of zinc concentrations. To each member of the dilution line, EDTA·2Na salt was added as a interfering chelating substance in a concentration of 200 mg/dl. The zinc content of each sample was measured using the following reagents:

### (Reagent 8)

0.5 M Acetate buffer solution (pH 6.5).

### (Reagent 9)

An aqueous solution in which 200 mg of 5-Br-PADAP being dissolved in purified water to a total volume of 500 ml.

### (Reagent 10)

0.5 M Acetate buffer solution containing 65 mM of lanthanum (pH 6.5).

### (Procedure)

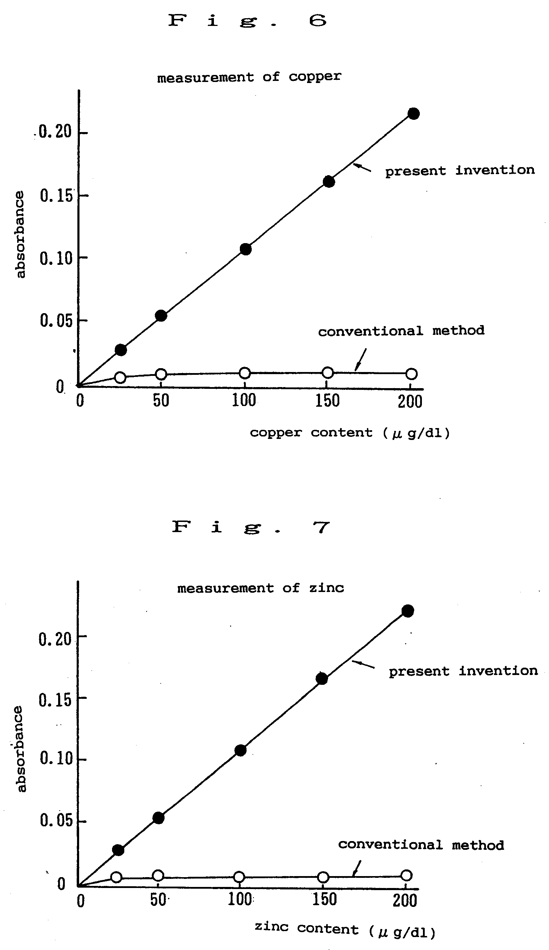

To 25 µl of each member of the dilution line containing EDTA, 2.0 ml of Reagent 8 was added, followed by heating to 37°C. Then, 200 µl of reagent 9 was further added to the resultant. After reaction of the resultant being carried out at 37°C for 5 minutes, the absorbance of each sample at 555 nm wave length was measured. Another test was carried out in the same manner as the above procedure except using Reagent 10 instead of Reagent 8. Further, another test was also carried out in the same manner as the above procedure except using purified water instead of the dilution series, and the resulting value was defined as a blank value. Results are shown in Fig. 7.

According to Fig. 7, it is evident that the method of the present invention can measure the zinc content more accurately compared to the conventional methods.

### Industrial Applicability

According to the present invention, the method for measuring metals in samples of body fluid, which comprises the above mentioned, can release the metals of interest bound to the co-existing substances in the samples from the co-existing substances easily. Therefore, even trace amounts of metals in samples of body fluid can be measured with high accuracy; the method of the present invention can be widely utilized in the filed of clinical examination.

## Claims

1. Colorimetric or enzymatic method to measure metals in a sample of body fluid containing interfering chelating substances, comprising the steps of:
(A) Adding a metal other than the metal of interest to the sample in an amount ranging from 5 µm to 2 M, said added metal releasing the metal of interest combined with interfering chelating substances in the sample from said interfering chelating substances;
(B) Adding a color-producing substance for specifically bonding to the metal of interest or adding an enzyme to the sample; and
(C) Spectroscopically detecting a signal derived from the color-producing substance or the activity of the enzyme.

2. The method according to claim 1, wherein said metals of interest are selected from the group consisting of magnesium, iron, zinc, potassium, calcium, manganese, aluminum, cadmium, chromium, mercury, copper, lead, nickel, selenium, bismuth, arsenic, sodium and phosphorus and the metals other than said metals of interest are different from said metals of interest and selected from the group consisting of lanthanum, manganese, terbium, yttrium, ytterbium, palladium, gallium, indium, strontium, aluminum, cobalt, bismuth and nickel.

## Patentansprüche

1. Kolorimetrisches oder enzymatisches Verfahren zum Messen von Metallen in einer Probe von Körperflüssigkeit, die störende komplexbildende Substanzen enthält, das die Schritte umfaßt von:
(A) Zugeben eines anderen Metalles als das Metall von Interesse zu der Probe in einer Menge im Bereich von 5 µM bis 2 M, wobei das zugegebene Metall das Metall von Interesse, das mit störenden, komplexbildenden Substanzen in der Probe kombiniert ist, von den störenden komplexbildenden Substanzen freisetzt;
(B) Zugeben einer farberzeugenden Substanz zum spezifischen Binden an das Metall von Interesse oder Zugeben eines Enzymes zu der Probe; und
(C) Spektroskopisches Nachweisen eines Signals, das von der farberzeugenden Substanz oder der Aktivität des Enzyms abgeleitet ist.

2. Verfahren gemäß Anspruch 1, bei dem die Metalle von Interesse aus der Gruppe ausgewählt sind, die aus Magnesium, Eisen, Zink, Kalium, Kalzium, Mangan, Aluminium, Kadmium, Chrom, Quecksilber, Kupfer, Blei, Nickel, Seien, Bismuth, Arsen, Natrium und Phosphor besteht und die anderen Metalle als die Metalle von Interesse von den Metallen von Interesse verschieden sind und aus der Gruppe ausgewählt sind, die aus Lanthan, Mangan, Terbium, Yttrium, Ytterbium, Palladium, Gallium, Indium, Strontium, Aluminium, Kobalt, Bismuth und Nickel besteht.

## Revendications

1. Procédé colorimétrique ou enzymatique pour mesurer les métaux dans un échantillon de fluide sanguin contenant des substances chélatantes interférentes comprenant les étapes :
(a) d'addition d'un métal, autre que le métal à mesurer, à l'échantillon, en une proposition de 5µm à 2 M, ledit métal ajouté libérant le métal à mesurer, combiné avec les substances chélatantes interférentes, dans l'échantillon à partir des substances chélatantes interférentes ;
(b) d'addition d'une substance générant une couleur pour la lier de façon spécifique au métal à mesurer ou d'addition d'un enzyme à l'échantillon ; et
(c) de détection spectroscopique d'un signal provenant de la substance générant une couleur ou de l'activité de l'enzyme.

2. Procédé selon la revendication 1 dans lequel les dits métaux à mesurer sont choisis dans le groupe constitué par le magnésium, le fer, le zinc, le potassium, le calcium, le manganèse, l'aluminium, le cadmium, le chrome, le mercure, le cuivre, le plomb, le nickel, le sélénium, le bismuth, l'arsenic, le sodium et le phosphore et dans lequel les autres métaux, différents des dits métaux à mesurer, sont choisis dans le groupe constitué par le lanthane, le manganèse, le terbium, l'yttrium, l'ytterbium, le palladium, le gallium, l'indium, le strontium, l'aluminium, le cobalt, le bismuth et le nickel.
